# FASCICULE DE BREVET EUROPEEN

(11) **EP 3 820 426 B1**
(45) Date de publication et mention de la délivrance du brevet: **30.03.2022**
(21) Numéro de dépôt: 19756422.2
(22) Date de dépôt: 11.07.2019
(51) Int. Cl.: A61H 33/06, A61F 7/00, A61H 33/00, A61H 35/00, A61H 33/14, A61F 7/02

(54) **APPAREIL DE TRAITEMENT AVEC UN SYSTEME DE CONTROLE DE L'ECOULEMENT DU MILIEU GAZEUX**
VORRICHTUNG MIT EINEM SYSTEM ZUR STEUERUNG DER STRÖMUNG DES GASFÖRMIGEN MEDIUMS
APPARATUS WITH A SYSTEM FOR CONTROLLING THE FLOW OF THE GASEOUS MEDIUM

(30) Priorité: 12.07.2018 FR 1856414
(43) Date de publication de la demande: 19.05.2021
(73) Titulaire: DTAMedical, 39300 Loulle (FR)
(72) Inventeur: DUFAY, François, 39300 LOULLE (FR)
(74) Mandataire: Cabinet Beau de Loménie
(86) Numéro de dépôt international: PCT/FR2019/051745
(87) Numéro de publication internationale: WO 2020/012131

(56) Documents cités:
- EP-A1- 2 260 825
- FR-A1- 3 052 662
- US-A- 4 224 941
- US-A1- 2005 191 372

## Description

La présente invention concerne le domaine technique du traitement corporel, topique ou systémique, curatif ou préventif, pour des applications thérapeutiques, esthétiques et cosmétiques pour l'homme et l'animal et plus particulièrement le traitement des tissus iésés, avec ou sans effraction, avec ou sans perte de substance.

Pour traiter les tissus lésés ou les plaies, l'état de la technique a proposé d'y apposer des pansements, ce qui pose les problèmes du contact entre le pansement et la plaie et les risques de prolifération microbienne.

Afin d'éviter ces problèmes, l'art antérieur a proposé différents appareils de traitement visant à exposer les tissus lésés à un milieu gazeux. Par exemple, le brevet EP 2 309 954 décrit un appareil de traitement comportant une enveloppe apte à être placée sur ou autour d'une partie à traiter du corps d'une personne. Cette enveloppe est munie d'une entrée pour un milieu gazeux et d'une sortie pour l'évacuation du milieu gazeux présent dans l'enveloppe.

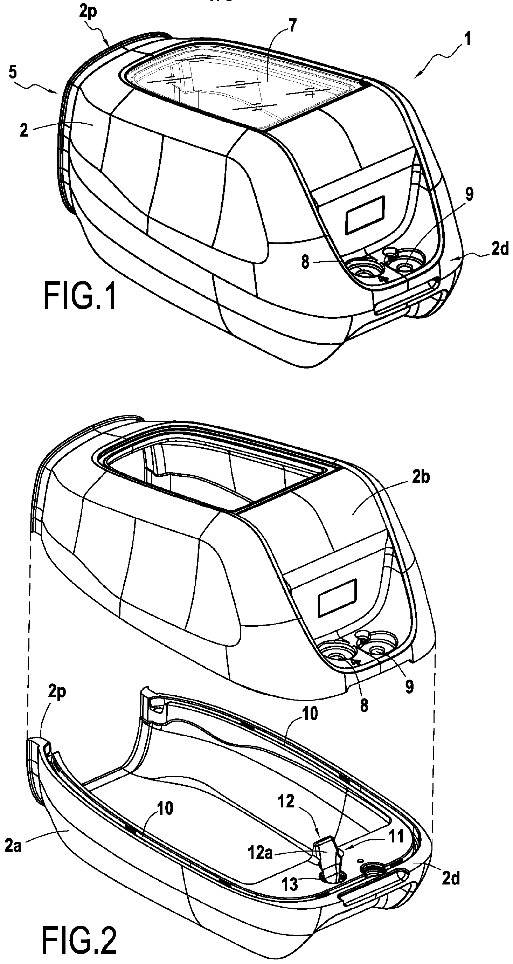

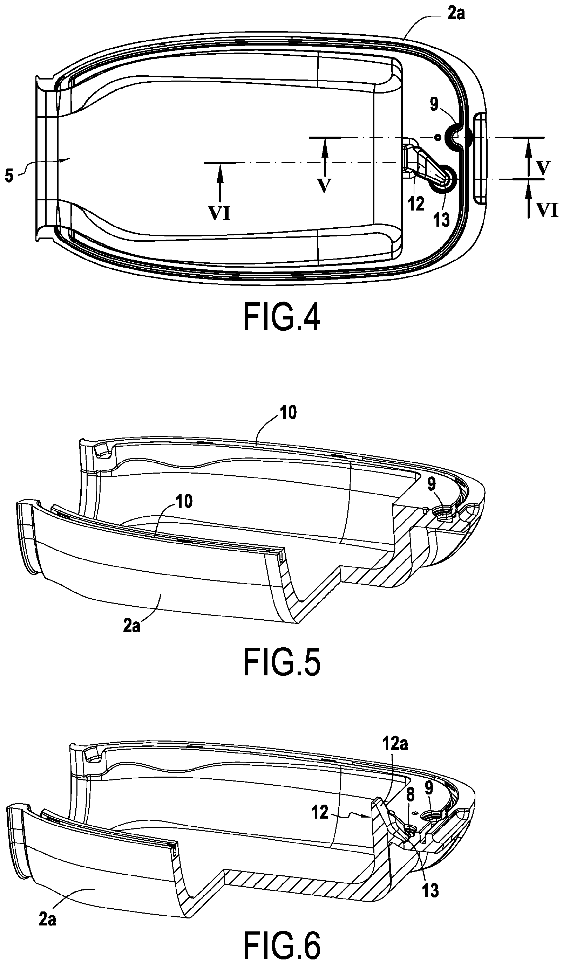

La demande de brevet US 2005/191372 décrit un dispositif de traitement au monoxyde d'azote gazeux comprenant une chambre, une source de monoxyde d'azote, une valve de contrôle et une pompe. La chambre peut être de toute forme et réalisée en tout matériau, et comprend une entrée et une sortie pour le gaz. De préférence, cette entrée et cette sortie sont disposées à distance l'une de l'autre afin d'augmenter le temps de passage du gaz dans la chambre. Un système de contrôle permet de contrôler l'écoulement gazeux au niveau de la valve et au niveau de la pompe. Selon le mode de réalisation représenté à la **Fig. 2**, une buse est fixée au niveau de l'entrée afin de diriger le monoxyde d'azote sur la zone à traiter. Selon le mode de réalisation représenté à la **Fig. 4**, la chambre comporte en outre un agitateur pour créer des conditions de turbulence à l'intérieur de ladite chambre. L'agitateur permet ainsi de renouveler le monoxyde d'azote au niveau de la zone à traiter.

Le brevet FR 3 052 662 décrit un système de génération d'un mélange gazeux pour une enceinte de traitement d'une plaie comprenant un générateur de mélange gazeux, une enceinte de traitement, un dispositif d'adjonction d'un gaz additionnel et/ou d'un aérosol, et une unité de contrôle. L'enceinte de traitement est agencée pour être disposée sur et/ou autour d'une partie du corps d'un patient de manière à engendrer une zone de traitement. L'enceinte de traitement comporte une entrée et une sortie pour le mélange gazeux. L'unité de contrôle permet de contrôler le générateur de mélange gazeux et de définir un débit, une température et composition du mélange gazeux à délivrer.

La demande de brevet EP 2 260 825 décrit un dispositif de génération d'un mélange gazeux, composé de gaz et de liquide, pour traiter un patient. Selon un premier mode de réalisation, ce dispositif comporte un moyen de couverture de la zone à traiter avec une entrée et une sortie pour le mélange gazeux, une partie adhésive, des sources de gaz et de liquide, et un moyen de contrôle de la pression et de la température notamment. L'entrée de mélange gazeux peut comprendre une valve pour un effet anti-retour.

Le brevet US 4 224 941 décrit un dispositif pour administrer un mélange gazeux sur la peau d'un patient comprenant une compresse adhésive, une pièce cylindrique liée à la compresse adhésive par l'un de ses côté et liée à un sac par l'autre de ses côtés, une entrée et une sortie pour le mélange gazeux situées sur ledit sac. L'entrée est reliée à une source de mélange gazeux via un connecteur.

Il ressort des documents de l'art antérieur qu'un appareil qui expose les tissus lésés à un milieu gazeux est associé à un dispositif permettant de contrôler l'atmosphère régnant à l'intérieur de l'enveloppe. En particulier, la température doit être contrôlée car la fenêtre de température du traitement est généralement étroite. Par ailleurs, les tissus ne peuvent pas être exposés longtemps à des températures élevées. De plus, dans les domaines thérapeutiques, il est important de contrôler la concentration en oxygène afin de limiter, voire annuler la prolifération bactérienne aérobie ou anaérobie.

L'hygrométrie du milieu gazeux injecté est également contrôlée afin d'optimiser la régénération des tissus lésés. D'une manière générale, les paramètres tels que la température, l'hygrométrie et la composition du milieu gazeux sont contrôlés de manière à optimiser le traitement à réaliser sur les tissus lésés.

En pratique, malgré le contrôle des divers paramètres du milieu gazeux injecté à l'intérieur de l'enveloppe, il a été constaté que l'évolution des tissus lésés ne correspondait pas à l'évolution attendue par le traitement appliqué. Outre une telle distorsion dans les résultats attendus, le patient ressent fréquemment une gêne ou un inconfort pendant la réalisation du traitement.

Le demandeur a eu le mérite de mettre en évidence que les déficiences obtenues dans le traitement ne proviennent pas des paramètres retenus mais sont liées aux conditions d'application du milieu gazeux à l'intérieur de l'enveloppe de traitement. En effet, il a été constaté que l'atmosphère en contact avec les tissus lésés et régnant à l'intérieur des enveloppes de traitement connues présente des paramètres dont les valeurs ne correspondent pas aux valeurs retenues ou de consigne.

La présente invention vise à remédier aux inconvénients de l'état de la technique en proposant un appareil de traitement selon la revendication visant à exposer dans une enveloppe, des tissus lésés à un milieu gazeux et conçu de manière que les conditions d'application du milieu gazeux permettent d'optimiser le traitement réalisé.

Pour atteindre un tel objectif, l'appareil de traitement conforme à l'invention comporte une enveloppe apte à être placée sur ou autour d'une partie du corps d'une personne de sorte que l'enveloppe présente une zone de positionnement pour ladite partie du corps de 1a personne, l'enveloppe étant munie d'une entrée pour un milieu gazeux et d'une sortie pour l'évacuation du milieu gazeux présent dans l'enveloppe. Selon l'invention, cet appareil comporte un système de contrôle de l'écoulement du milieu gazeux pour diriger le milieu gazeux pénétrant dans l'enceinte par l'entrée, en dehors de la zone de positionnement et pour homogénéiser le milieu gazeux présent dans l'enveloppe.

L'appareil de traitement selon l'invention empêche l'arrivée directe du milieu gazeux sur la peau du patient notamment sur les tissus lésés, ce qui évite la dessiccation de la plaie et permet la cicatrisation car l'assèchement de la plaie empêche cette cicatrisation.

L'appareil selon l'invention assure une homogénéisation du milieu gazeux à l'intérieur de l'enveloppe avant son contact avec les tissus lésés. L'écoulement du milieu gazeux dans l'enveloppe, selon un régime tourbillonnaire permet d'homogénéiser l'atmosphère présente à l'intérieur de l'enveloppe. Cette homogénéisation permet que les paramètres de l'atmosphère en contact avec les tissus lésés possèdent des valeurs correspondant aux valeurs retenues ou de consigne, cette homogénéisation étant obtenue quelles que soient les variations de ces paramètres.

La mise en œuvre de ces conditions d'application du milieu gazeux au sein de l'enveloppe de traitement fait que le patient ne ressent aucune gêne ou inconfort pendant la réalisation du traitement.

Selon une caractéristique de l'invention, le système de contrôle de l'écoulement comporte un déflecteur modifiant la direction d'écoulement du milieu gazeux dans l'enveloppe, en dehors de la zone de positionnement et créant un régime tourbillonnaire.

Selon un premier exemple de réalisation, le déflecteur présente une surface d'orientation du milieu gazeux en direction de la surface interne de l'enveloppe et sur laquelle est dirigé le milieu gazeux pénétrant dans l'enveloppe par l'entrée.

Selon un deuxième exemple de réalisation, le déflecteur comporte une canalisation coudée montée sur l'entrée. Avantageusement, la canalisation coudée est montée mobile en rotation autour de son axe pour régler l'orientation de l'écoulement.

Selon une variante préférée de réalisation, l'enveloppe de l'appareil de traitement conforme à l'invention est munie d'un dispositif de visualisation de la zone de positionnement pour ladite partie du corps de la personne et le système de contrôle de l'écoulement dirige le milieu gazeux en direction du dispositif de visualisation pour assurer son désembuage.

En effet, le niveau de l'hygrométrie du milieu gazeux et la différence de température entre l'intérieur et l'extérieur de l'enceinte créent les conditions favorables pour l'apparition d'une buée notamment sur le dispositif de visualisation.

La canalisation du milieu gazeux vers le système de visualisation permet d'éviter ou de supprimer la présence de buée de sorte que le suivi thérapeutique peut être mené à bien tout au long du traitement, quelles que soient les températures à l'intérieur et à l'extérieur de l'enveloppe.

Il est à noter que l'appareil de traitement selon l'invention peut comporter en tant que dispositif de visualisation, un hublot aménagé dans l'enveloppe ou un système optique avec ou sans prise d'images.

De plus, l'appareil de traitement selon l'invention peut comporter en outre en combinaison au moins l'une et/ou l'autre des caractéristiques additionnelles suivantes :
- l'entrée et la sortie sont positionnées côte à côte selon des directions divergentes ;
- l'entrée et la sortie sont aménagées sur un corps amovible par rapport à l'enveloppe.

L'appareil de traitement conforme à l'invention vise à exposer les tissus lésés à un milieu gazeux. De façon complémentaire, l'appareil de traitement conforme à l'invention est adapté pour incorporer un liquide dans l'enveloppe de traitement afin de faciliter le lavage de la plaie et/ou d'éliminer les substrats exsudatifs provenant de celle-ci. Cette méthode est appelée instillation. Selon une autre application, le liquide injecté dans l'enveloppe de traitement peut être un médicament qui est directement mis en contact avec la plaie dans un cadre thérapeutique.

A cet effet, l'entrée de l'enveloppe de traitement est adaptée pour être raccordée à une source d'alimentation en milieu liquide et l'enveloppe de traitement est pourvue d'une sortie d'évacuation pour le milieu liquide correspondant à la sortie du milieu gazeux ou à une sortie complémentaire aménagée sur l'enveloppe de traitement.

L'appareil de traitement conforme à l'invention vise à exposer les tissus lésés à un milieu gazeux mais également à un milieu liquide.

Un autre objet ne faisant pas partie de l'invention est de proposer un procédé de traitement de tissus lésés à l'aide d'un appareil de traitement comportant une enveloppe placée sur ou autour d'une partie du corps d'une personne et munie d'une entrée pour un milieu gazeux et d'une sortie pour l'évacuation du milieu gazeux présent dans l'enveloppe.

Le procédé consiste à contrôler l'écoulement du milieu gazeux pour diriger le milieu gazeux en dehors de la zone dans laquelle est positionnée ladite partie du corps de la personne et pour homogénéiser le milieu gazeux présent dans l'enveloppe.

Avantageusement, le procédé consiste à assurer l'écoulement du milieu gazeux dans l'enveloppe selon un régime tourbillonnaire.

De préférence, le procédé consiste à diriger le milieu gazeux en direction d'un dispositif de visualisation de la partie du corps de la personne pour assurer le désembuage du dispositif de visualisation.

Selon un procédé complémentaire, ne faisant pas partie de l'invention, un milieu liquide est injecté par l'entrée de l'enveloppe et le milieu liquide est évacué de l'enveloppe par la sortie d'évacuation du milieu gazeux ou par une sortie complémentaire spécifique pour le milieu liquide.

Selon un exemple de mise en œuvre, le procédé consiste à orienter une canalisation coudée montée sur l'entrée de l'enceinte pour injecter le milieu liquide à l'intérieur de la zone dans laquelle est positionnée la partie du corps de la personne.

Diverses autres caractéristiques ressortent de la description faite ci-dessous en référence aux dessins annexés qui montrent, à titre d'exemples non limitatifs, des formes de réalisation de l'objet de 'invention conformément aux revendications.
La **Figure 1** est une vue en perspective d'un exemple de réalisation d'un appareil de traitement conforme à l'invention.
La **Figure 2** est une vue en perspective éclatée de l'exemple de réalisation de l'appareil de traitement illustré à la **Fig. 1****.**
La **Figure 3** est une vue en coupe élévation de l'appareil de traitement conforme à l'invention, adapté pour le traitement d'une partie de la jambe d'une personne.
La **Figure 4** est une vue de dessus de la partie inférieure de l'appareil de traitement conforme à l'invention.
Les **Figures 5** et **6** sont des vues en perspective coupées partiellement selon les lignes respectivement V-V et VI-VI, montrant la partie inférieure de l'appareil de traitement conforme à l'invention.
La **Figure** 7 montre d'autres exemples de réalisation d'un appareil de traitement conforme à l'invention.
La **Figure 8** illustre un autre mode de réalisation d'un appareil de traitement conforme à l'invention, mettant en œuvre un milieu gazeux et un milieu liquide.

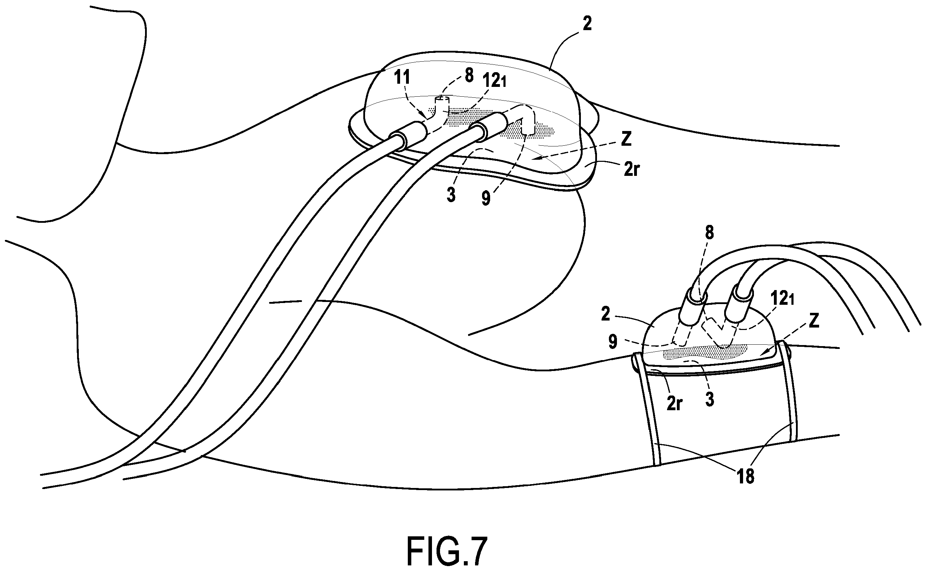

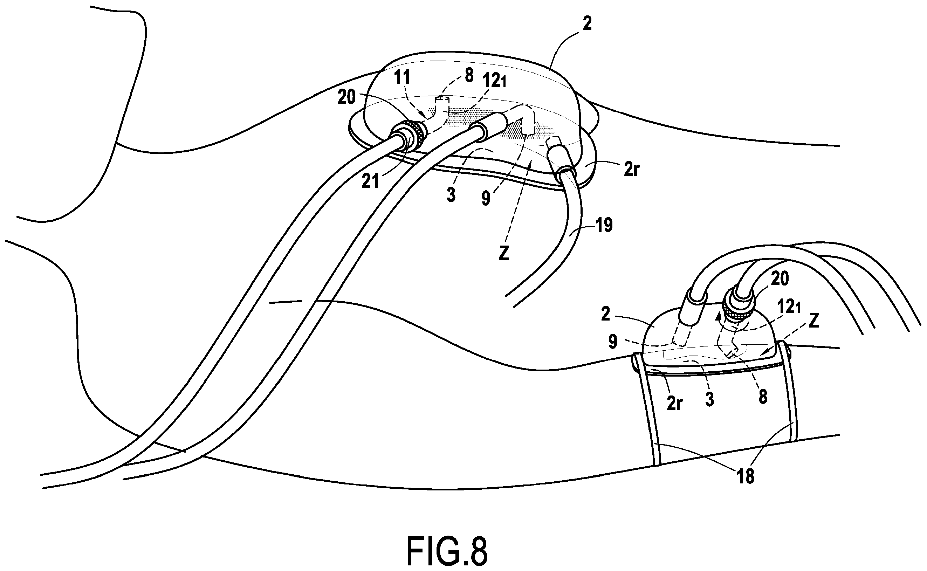

Les **Figures** représentent un appareil **1** conforme à l'invention comportant une enveloppe de traitement **2,** apte à être placée sur ou autour d'une partie **3** du corps d'une personne, pourvue des tissus lésés à traiter par un milieu gazeux au sens général. Dans l'exemple illustré aux **Fig. 1** à **6****,** l'enveloppe de traitement **2** est destinée être placée autour d'une partie du corps d'une personne à traiter et en particulier, une jambe **3** d'une personne. Les **Fig. 7** et **8** illustrent un autre exemple de réalisation pour lequel l'enveloppe de traitement **2** est apte à être placée sur une partie **3** du corps d'une personne, tel que le bras ou le thorax d'une personne.

Bien entendu, l'appareil de traitement **1** conforme à l'invention comporte une enveloppe **2** dont la forme et les dimensions sont adaptées à la partie **3** du corps à traiter. Quelle que soit la partie **3** du corps à traiter, l'enveloppe de traitement **2** présente une zone de positionnement **Z** pour assurer que les tissus lésés de ladite partie du corps de la personne puissent être en contact avec le milieu gazeux présent dans cette enveloppe de traitement **2.** Il doit être compris que la zone de positionnement **Z** correspond au volume occupé par les tissus lésés à traiter et éventuellement par une partie du corps qui n'est pas à traiter et située autour ou dans le prolongement des tissus lésés. Le dimensionnement de l'enveloppe de traitement **2** tient compte de cette zone de positionnement **Z** de manière que lorsque cette zone **Z** est occupée par la partie **3** du corps à traiter, l'enveloppe présente un volume pour le milieu gazeux, adapté pour assurer le traitement des tissus lésés.

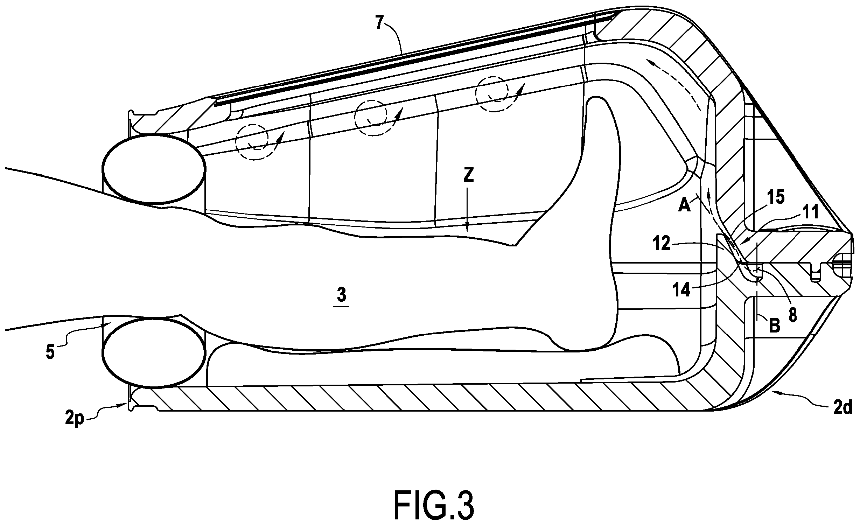

Dans l'exemple illustré aux **Fig. 1** à **6****,** l'enveloppe de traitement **2** se présente sous la forme d'un caisson allongé, ouvert à une extrémité pour le passage du bras ou de la jambe tandis que dans l'exemple illustré aux **Fig. 7** et **8****,** l'enveloppe de traitement **2** se présente sous la forme d'une cloche venant en appui sur une partie du corps d'une personne. Pour l'exemple illustré aux **Fig. 1** à **6**, la zone de positionnement **Z** pour la partie **3** du corps à traiter, se trouve située dans le volume interne de l'enveloppe de traitement **2** et à distance de cette enveloppe et correspond au volume occupé par la jambe comme illustré à la **Fig. 3****.** Dans l'exemple illustré aux **Fig. 7** et **8**, cette zone de positionnement **Z** qui vient fermer l'enveloppe de traitement **2**, s'étend à l'opposé ou en regard de l'enveloppe de traitement et correspond à une surface du thorax ou une surface du bras.

Dans l'exemple illustré aux **Fig. 1** à **6****,** l'enveloppe de traitement **2** se présente sous la forme d'un caisson rigide constitué par exemple d'une coque inférieure **2a** et d'une coque supérieure **2b** destinées à être assemblées ensemble pour former par sa paroi, une enceinte fermée accessible par un passage **5** pour l'introduction de la partie **3** du corps à traiter. L'enveloppe allongée **2** présente ainsi une extrémité proximale **2p** dans laquelle est aménagé le passage **5** et une extrémité distale **2d** située à l'opposé de l'extrémité proximale **2p.** Dans l'exemple illustré aux **Fig. 7** et **8****,** l'enveloppe de traitement **2** se présente sous la forme d'une coque de forme hémisphérique.

Selon une variante préférée de réalisation, l'enveloppe **2** est munie d'un dispositif **7** de visualisation de la zone de positionnement **Z** pour ladite partie **3** du corps de la personne. Selon l'exemple illustré aux **Fig. 1** à **6****,** l'enveloppe **2** comporte en tant que dispositif de visualisation **7,** un hublot aménagé sur la coque supérieure **2b** et plus précisément sur la face supérieure de la coque supérieure **2b.** La présence du hublot **7** permet d'observer l'état des tissus lésés et de surveiller leur évolution. Dans l'exemple illustré aux **Fig. 7** et **8****,** l'enveloppe **2** est réalisée avec un matériau transparent ou translucide pour former le dispositif de visualisation. Bien entendu, il peut être envisagé d'aménager un hublot sur l'enceinte en forme de cloche illustrée aux **Fig. 7** et **8**.

L'enveloppe de traitement **2** peut être équipée d'un dispositif de visualisation **7** de nature différente d'une vitre comme illustré sur les dessins. Par exemple, le dispositif de visualisation peut être réalisé par un système optique avec ou sans prise d'images tel qu'un capteur CCD, un endoscope, une caméra ou une fibre optique.

Classiquement, l'enveloppe de traitement **2** est munie d'une entrée **8** pour un milieu gazeux et d'une sortie **9** pour l'évacuation du milieu gazeux présent dans l'enveloppe **2.** L'entrée **8** permet l'introduction à l'intérieur de l'enveloppe de traitement **2** d'un milieu gazeux de toute nature appropriée tel que par exemple de l'air enrichi ou appauvri en oxygène. Typiquement, l'entrée **8** est reliée à un circuit externe connecté à une machine de traitement non représentée mais connue en soi permettant d'injecter à l'intérieur de l'enveloppe de traitement, un milieu gazeux dont les différents paramètres sont contrôlés comme par exemple, la température, l'hygrométrie, la concentration en oxygène ou la pression.

Dans l'exemple illustré aux **Fig. 1** à **6****,** l'entrée **8** débouche à l'extérieur de l'enveloppe de traitement, sur la coque supérieure **2b,** au niveau de l'extrémité distale **2d,** pour être reliée au circuit externe connecté à la machine de traitement.

La sortie **9** est reliée, par un circuit externe non représenté, à un système d'aspiration permettant d'évacuer une partie de l'atmosphère contenue dans l'enveloppe de traitement **2** afin de créer une circulation dans l'enveloppe de traitement **2.** La sortie **9** est aménagée de manière à extraire l'atmosphère homogène régnant dans l'enveloppe et non pas le milieu gazeux venant de pénétrer par l'entrée **8.** Dans l'exemple illustré aux **Fig. 1** à **6****,** la sortie **9** est réalisée par un conduit **10** aménagé dans chacune des parois latérales de l'enveloppe de traitement, dont l'entrée est située au niveau l'extrémité proximale **2p** et débouche par une sortie commune à l'extérieur de l'enveloppe de traitement **2,** sur la coque supérieure **2b,** au niveau de l'extrémité distale **2d.** Selon cette variante de réalisation, l'entrée **8** et la sortie **9** débouchent côte à côte, à l'extérieur de l'enveloppe de traitement, sur la coque supérieure **2b.**

Conformément à l'invention, l'appareil **1** comporte un système de contrôle **11** de l'écoulement du milieu gazeux à l'intérieur de l'enveloppe de traitement **2,** adapté pour diriger le milieu gazeux pénétrant dans l'enveloppe par l'entrée **8,** en dehors de la zone de positionnement **Z** et pour homogénéiser le milieu gazeux présent dans l'enveloppe **2.** L'enveloppe de traitement **2** est donc équipée du système **11** permettant de contrôler l'écoulement du milieu gazeux à l'intérieur de l'enveloppe de traitement **2.**

Le système de contrôle de l'écoulement **11** dirige le milieu gazeux en dehors de la zone de positionnement **Z** évitant ainsi l'arrivée directe avec une vitesse importante, du milieu gazeux sur la peau du patient notamment sur les tissus lésés. La canalisation du milieu gazeux en dehors de la zone de positionnement **Z** c'est-à-dire en dehors de ladite partie du corps de la personne à traiter, évite la dessiccation de la plaie et permet sa cicatrisation. Tel que cela sera expliqué dans la suite de la description, le système de contrôle de l'écoulement **11** canalise le milieu gazeux en particulier en direction de l'enveloppe de traitement **2.** Le milieu gazeux qui pénètre par l'entrée **8** est ainsi dirigé directement en direction de l'enveloppe.

Le système de contrôle de l'écoulement **11** assure également une homogénéisation du milieu gazeux à l'intérieur de l'enveloppe avant son contact avec les tissus lésés. Le système de contrôle de l'écoulement **11** assure l'écoulement du milieu gazeux dans l'enveloppe de traitement **2** selon un régime tourbillonnaire permettant d'homogénéiser l'atmosphère présente à l'intérieur de l'enveloppe. L'écoulement du milieu gazeux selon un régime tourbillonnaire et en dehors de la zone de positionnement **Z** permet d'obtenir une atmosphère homogène avant son contact avec les tissus lésés. Il en résulte que l'atmosphère en contact avec les tissus lésés respecte les paramètres souhaités.

Le système de contrôle **11** de l'écoulement du milieu gazeux peut être réalisé de toute manière appropriée pour diriger le milieu gazeux, en dehors de la zone de positionnement **Z** et pour homogénéiser le milieu gazeux présent dans l'enveloppe **2.** D'une manière générale, le système de contrôle de l'écoulement **11** comporte un déflecteur modifiant la direction d'écoulement du milieu gazeux dans l'enveloppe **2**, en dehors de la zone de positionnement **Z** et créant un régime tourbillonnaire.

Dans l'exemple de réalisation illustré sur les **Fig. 1** à **6****,** le système de contrôle **11** comporte un déflecteur **12** sur lequel est dirigé le milieu gazeux pénétrant dans l'enveloppe de traitement par l'entrée **8.** Le milieu gazeux pénètre à l'intérieur de l'enveloppe de traitement **2** avec une vitesse adaptée pour assurer un régime d'écoulement tourbillonnaire. Typiquement, le milieu gazeux possède à l'entrée **8** une vitesse suffisante pour permettre au flux entrant de se propager dans tout le volume de l'enceinte de traitement.

Comme cela apparaît plus précisément aux **Fig. 2****,** **4** et **6****,** l'entrée **8** comporte un puits **13** aménagé dans la coque supérieure **2b** et débouchant à l'extérieur de l'enveloppe de traitement. Ce puits **13** est également aménagé dans la coque inférieure **2a,** en se prolongeant par un conduit de déflexion **14** débouchant à l'intérieur de l'enveloppe de traitement **2.** Ce conduit de déflexion **14** est délimité entre le déflecteur **12** et la surface interne **15** de la coque supérieure **2b.** Le déflecteur **12** est aménagé à l'intérieur de l'enveloppe de traitement **2,** au niveau de son extrémité distale **2d.**

Selon une caractéristique avantageuse de réalisation, le déflecteur **12** présente une surface d'orientation **12a** du milieu gazeux en direction de la surface interne de l'enveloppe de traitement. Dans l'exemple illustré, le déflecteur **12** dirige le milieu gazeux afin que son écoulement suive la surface interne de la coque supérieure **2b,** de sa partie inférieure à sa partie supérieure et de sa partie distale **2d** en direction de sa partie proximale **2p.**

Avantageusement, le déflecteur **12** présente une surface d'orientation **12a** située en vis-à-vis de la paroi **15** de l'enveloppe de traitement et délimitant le conduit de déflexion **14.** Cette surface d'orientation **12a** possède un axe d'extension **A** faisant avec l'axe **B** de l'entrée **8**, un angle compris entre 10 et 170°.

Selon une autre caractéristique avantageuse de réalisation, cette surface d'orientation **12a** du déflecteur est plane ou incurvée avec ou non des bords relevés.

Dans le cas où l'enveloppe de traitement **2** est équipée d'un dispositif de visualisation **7,** le système de contrôle de l'écoulement **11** dirige le milieu gazeux en direction du dispositif de visualisation pour assurer son désembuage. Tel que cela ressort des **Fig. 1** à **6**, l'écoulement de milieu gazeux à partir de l'entrée **8** est dirigé de manière à venir lécher le hublot **7** et assurer son désembuage.

Il ressort de la description qui précède que le milieu gazeux qui pénètre à l'extrémité distale **2d** de l'enveloppe de traitement **2** est canalisé en direction de la paroi de la coque supérieure **2b,** de sa partie inférieure jusqu'à sa partie supérieure, en se dirigeant vers la partie proximale **2p** de l'enveloppe de traitement, en présentant un écoulement tourbillonnaire comme représenté à la **Fig. 3****.** L'extraction du milieu gazeux présent dans l'enveloppe de traitement **2** est réalisée au niveau de la partie proximale **2p,** par les conduits **10** reliés à la sortie **9.**

Dans l'exemple de réalisation illustré aux **Fig. 7** et **8**, le système de contrôle de l'écoulement **11** comporte en tant que déflecteur, une canalisation ou conduite coudée **12₁** d'amenée du milieu gazeux montée sur l'entrée **8** pour diriger le milieu gazeux en dehors de la zone de positionnement **Z** et pour homogénéiser le milieu gazeux présent dans l'enveloppe **2.** Selon cet exemple de réalisation, l'enveloppe de traitement **2** se présente sous la forme d'un caisson creux de forme allongée ou hémisphérique, pourvu d'un rebord **2r** venant en appui sur le corps de la personne. Ce rebord **2r** peut être utilisé pour permettre la fixation de l'appareil sur le corps à l'aide par exemple d'une sangle ou d'un brassard **18.**

L'enveloppe de traitement **2** est pourvue d'une entrée **8** équipée d'une canalisation ou d'une conduite **12₁** coudée, montée pour diriger le milieu gazeux en dehors de la zone de positionnement **Z** et pour homogénéiser le milieu gazeux présent dans l'enveloppe **2.** La zone de positionnement **Z** s'étendant au niveau du plan passant par le rebord d'appui de l'enveloppe de traitement, l'entrée **8** est aménagée à proximité de ce rebord avec la canalisation dirigée en direction de la paroi de l'enveloppe c'est-à-dire en direction opposée du plan passant par le rebord d'appui **2r.** La canalisation ou conduite **12₁** coudée délivre le milieu gazeux avec une vitesse adaptée pour obtenir un écoulement tourbillonnaire à l'intérieur de l'enveloppe de traitement **2.**

La canalisation ou conduite **12₁** coudée est adaptée pour diriger le milieu gazeux en direction du dispositif de visualisation constitué dans l'exemple illustré aux **Fig. 7** et **8**, par tout ou partie de l'enveloppe de traitement **2** conçue pour former un hublot.

Selon une caractéristique avantageuse de réalisation, l'entrée **8** et la sortie **9** sont positionnées côte à côte mais selon des directions divergentes pour permettre une circulation du milieu gazeux à l'intérieur de l'enveloppe de traitement **2.** Selon une telle disposition, l'entrée **8** et la sortie **9** sont aménagées sur un corps non représenté, monté de manière amovible par rapport à l'enveloppe de traitement **2.** Ainsi, cette entrée **8** et la sortie **9** peuvent être montées sur différentes enveloppes de traitement **2** présentant des formes adaptées aux parties du corps à traiter.

Dans le cas où l'appareil de traitement comporte un dispositif de visualisation de la partie du corps de la personne, le procédé consiste à diriger le milieu gazeux en direction d'un dispositif de visualisation de la partie du corps de la personne pour assurer le désembuage du dispositif de visualisation.

Il ressort de la description qui précède, que l'appareil de traitement **1** conforme à l'invention permet d'exposer dans une enveloppe, des tissus lésés à un milieu gazeux en permettant que les conditions d'application du milieu gazeux optimisent le traitement réalisé. Selon un mode de réalisation avantageux, l'appareil de traitement **1** conforme à l'invention permet également d'incorporer dans l'enveloppe de traitement **2,** un milieu liquide de tous types tels un liquide physiologique dans le cadre du nettoyage d'une plaie ou un liquide actif médicamenteux dans le cas d'un traitement spécifique.

Selon ce mode de réalisation illustré à la **Fig. 8****,** l'entrée **8** de l'appareil de traitement **1** est adaptée pour être raccordée à une source d'alimentation en milieu liquide. L'injection du milieu liquide peut être réalisée à l'aide du circuit externe utilisé pour l'injection du milieu gazeux et connecté également à cette source d'alimentation en milieu liquide. L'injection du milieu liquide peut être réalisée à l'aide d'un circuit spécifique communiquant avec une source d'alimentation en milieu liquide et raccordé, en amont de l'entrée **8**, sur le circuit externe utilisé pour l'injection du milieu gazeux.

L'enveloppe **2** est pourvue d'une sortie d'évacuation pour le milieu liquide correspondant soit à la sortie **9** du milieu gazeux comme illustré sur les dessins par l'enveloppe portée par le bras d'une personne ou soit à une sortie complémentaire **19** spécifique pour l'extraction du milieu liquide. Cette sortie complémentaire **19** est bien entendu placée à l'altitude minimale dans l'enveloppe comme illustré sur les dessins par l'enveloppe portée par le thorax d'une personne. Bien entendu, l'enveloppe **2** est montée de manière étanche sur une partie du corps d'une personne à l'aide de la sangle ou du brassard **18** et/ou d'un cordon d'étanchéité rapporté au niveau du rebord **2r.**

Selon une caractéristique avantageuse de ce mode de réalisation, la canalisation ou conduite coudée **12₁** formant le déflecteur est montée mobile en rotation autour de son axe pour régler l'orientation de l'écoulement à l'intérieur de l'enveloppe **2.** Cette canalisation ou conduite coudée **12₁** peut être manœuvrée à partir de l'extérieur de l'enveloppe par tous moyens appropriés tels qu'une bague moletée **20** montée solidaire d'un prolongement de la canalisation coudée **12₁** faisant saillie hors de l'enveloppe. Cette bague moletée **20** est guidée en rotation sur un raccord **21** monté de manière fixe et étanche sur l'enveloppe.

A l'aide de la bague moletée **20,** l'entrée **8** peut être orientée soit en position haute (vers le hublot) soit en position basse (vers la plaie), selon le substrat fluidique utilisé (liquide ou gaz) par l'utilisateur. Il est à noter que le prolongement de la canalisation ou conduite coudée **12₁** peut présenter un caractère télescopique pour être allongée dans sa longueur afin de faciliter l'orientation de l'entrée **8.**

Lorsqu'un liquide (médicamenteux et/ou de lavage ...) est administré, l'appareil de traitement selon l'invention permet de diriger le flux de liquide sur la plaie afin de faciliter le nettoyage de la plaie ou d'y apporter des éléments thérapeutiques ou anti bactériens. L'entrée **8** du milieu gazeux peut ainsi être utilisée pour l'injection de liquide de tous types : liquide physiologique dans le cadre du nettoyage d'une plaie, liquide actif médicamenteux dans le cas d'un traitement spécifique.

L'appareil selon l'invention offre la mise en œuvre d'un procédé de traitement de tissus lésés consistant à contrôler l'écoulement du milieu gazeux pour diriger le milieu gazeux en dehors de la zone **Z** dans laquelle est positionnée ladite partie du corps de la personne mais également à injecter par l'entrée **8** de l'enveloppe, un milieu liquide et à assurer l'évacuation de l'enveloppe d'un milieu liquide, par la sortie d'évacuation **9** du milieu gazeux ou par une sortie complémentaire **19.** Le procédé qui ne fait pas partie de l'invention, consiste avantageusement à orienter la canalisation ou conduite coudée **12₁** montée sur l'entrée **8** pour injecter le milieu liquide à l'intérieur de la zone **Z** dans laquelle est positionnée la partie du corps de la personne présentant les tissus lésés.

## Revendications

1. - Appareil de traitement de tissus lésés comportant :
- une enveloppe **(2)** apte à être placée sur ou autour d'une partie du corps d'une personne de sorte que l'enveloppe **(2)** présente une zone de positionnement **(Z)** pour ladite partie du corps de la personne, l'enveloppe **(2)** étant munie d'une entrée **(8)** pour un milieu gazeux et d'une sortie **(9)** pour l'évacuation du milieu gazeux présent dans l'enveloppe,
- un système de contrôle **(11)** de l'écoulement du milieu gazeux, **caractérisé en ce que** le système de contrôle **(11)** comporte un déflecteur **(12, 12₁**), ledit déflecteur **(12, 12₁**) modifiant la direction d'écoulement du milieu gazeux pénétrant dans l'enveloppe par l'entrée **(8),** en dehors de la zone de positionnement **(Z)** et créant un régime tourbillonnaire pour homogénéiser le milieu gazeux présent dans l'enveloppe **(2).**

2. - Appareil de traitement selon la revendication précédente, **caractérisé en ce que** le déflecteur **(12)** présente une surface d'orientation **(12a)** du milieu gazeux en direction de la surface interne de l'enveloppe et sur laquelle est dirigé le milieu gazeux pénétrant dans l'enveloppe par l'entrée **(8).**

3. - Appareil de traitement selon la revendication 1, **caractérisé en ce que** le déflecteur (**12₁**) comporte une canalisation coudée montée sur l'entrée **(8).**

4. - Appareil de traitement selon la revendication 3, **caractérisé en ce que** le déflecteur (**12₁**) comporte une canalisation coudée montée mobile en rotation autour de son axe pour régler l'orientation de l'écoulement.

5. - Appareil de traitement selon l'une des revendications précédentes, **caractérisé en ce que** l'enveloppe **(2)** est munie d'un dispositif de visualisation **(7)** de la zone de positionnement **(Z)** pour ladite partie du corps de la personne et **en ce que** le système de contrôle de l'écoulement **(11)** dirige le milieu gazeux en direction du dispositif de visualisation **(7)** pour assurer son désembuage.

6. - Appareil de traitement selon l'une des revendications précédentes, **caractérisé en ce que** l'entrée **(8)** et la sortie **(9)** sont positionnées côte à côte selon des directions divergentes.

7. - Appareil de traitement selon la revendication précédente, **caractérisé en ce que** l'entrée **(8)** et la sortie **(9)** sont aménagées sur un corps amovible par rapport à l'enveloppe.

8. - Appareil de traitement selon l'une des revendications 5 à 7, **caractérisé en ce qu'**il comporte en tant que dispositif de visualisation **(7),** un hublot aménagé dans l'enveloppe ou un système optique avec ou sans prise d'images.

9. - Appareil de traitement selon l'une des revendications précédentes, **caractérisé en ce que** l'entrée **(8)** est adaptée pour être raccordée à une source d'alimentation en milieu liquide et **en ce que** l'enveloppe **(2)** est pourvue d'une sortie d'évacuation pour le milieu liquide correspondant à la sortie **(9)** du milieu gazeux ou à une sortie complémentaire **(19)** aménagée sur l'enveloppe.

## Patentansprüche

1. Gerät zur Behandlung von geschädigtem Gewebe, umfassend:
- ein Gehäuse (2), das geeignet ist, an einem oder um einen Teil des Körpers einer Person platziert zu werden, so dass das Gehäuse (2) einen Positionierungsbereich (Z) für den Teil des Körpers der Person aufweist, wobei das Gehäuse (2) mit einem Einlass (8) für ein gasförmiges Medium und mit einem Auslass (9) zum Abführen des in dem Gehäuse vorhandenen gasförmigen Mediums versehen ist,
- ein System zum Steuern (11) der Strömung des gasförmigen Mediums,
**dadurch gekennzeichnet, dass** das Steuersystem (11) einen Deflektor (12, 12₁) umfasst, wobei der Deflektor (12, 12₁) die Strömungsrichtung des durch den Einlass (8) in das Gehäuse eindringenden gasförmigen Mediums außerhalb des Positionierungsbereiches (Z) verändert und einen Wirbelbetrieb zum Homogenisieren des in dem Gehäuse (2) vorhandenen gasförmigen Mediums erzeugt.

2. Behandlungsgerät nach dem vorhergehenden Anspruch, **dadurch gekennzeichnet, dass** der Deflektor (12) eine Fläche zum Ausrichten (12a) des gasförmigen Mediums in Richtung der Innenfläche des Gehäuses aufweist, auf die das durch den Einlass (8) in das Gehäuse eindringende gasförmige Medium gerichtet ist.

3. Behandlungsgerät nach Anspruch 1, **dadurch gekennzeichnet, dass** der Deflektor (12₁) ein am Einlass (8) angebrachtes gebogenes Rohr umfasst.

4. Behandlungsgerät nach Anspruch 3, **dadurch gekennzeichnet, dass** der Deflektor (12₁) ein gebogenes Rohr aufweist, das um seine Achse herum drehbar gelagert ist, um die Ausrichtung der Strömung einzustellen.

5. Behandlungsgerät nach einem der vorhergehenden Ansprüche, **dadurch gekennzeichnet, dass** das Gehäuse (2) mit einer Vorrichtung zum Anzeigen (7) des Positionierungsbereiches (Z) für den Teil des Körpers der Person versehen ist und dass das System zum Steuern der Strömung (11) das gasförmige Medium in Richtung der Anzeigevorrichtung (7) leitet, um deren Beschlagfreihalten sicherzustellen.

6. Behandlungsgerät nach einem der vorhergehenden Ansprüche, **dadurch gekennzeichnet, dass** der Einlass (8) und der Auslass (9) in divergierenden Richtungen nebeneinander angeordnet sind.

7. Behandlungsgerät nach dem vorhergehenden Anspruch, **dadurch gekennzeichnet, dass** der Einlass (8) und der Auslass (9) an einem gegenüber dem Gehäuse lösbaren Körper angeordnet sind.

8. Behandlungsgerät nach einem der Ansprüche 5 bis 7, **dadurch gekennzeichnet, dass** es als Anzeigevorrichtung (7) ein in dem Gehäuse angeordnetes Sichtfenster oder ein optisches System mit oder ohne Bildaufnahme umfasst.

9. Behandlungsgerät nach einem der vorangehenden Ansprüche, **dadurch gekennzeichnet, dass** der Einlass (8) dazu ausgelegt ist, mit einer Quelle zur Versorgung mit flüssigem Medium verbunden zu werden, und dass das Gehäuse (2) mit einem Auslass zum Abführen für das flüssige Medium versehen ist, welcher dem Auslass (9) des gasförmigen Mediums oder einem ergänzenden Auslass (19), der an dem Gehäuse angeordnet ist, entspricht.

## Claims

1. A treatment apparatus of injured tissues comprising:
- a cover (2) capable of being placed over or around an individual's body part such that the cover (2) has a positioning zone (Z) for positioning said individual's body part, the cover (2) being equipped with an inlet (8) for a gaseous medium and with an outlet (9) for removing the gaseous medium present in the cover (2),
- a control system (11) of the flow of the gaseous medium,
**characterized in that** the control system (11) comprises a deflector (12, 12₁), said deflector (12, 12₁) modifying the direction of flow of the gaseous medium entering the cover via the inlet (8) away from the positioning zone (Z) and creating a turbulent flow regimen in order to homogenize the gaseous medium present inside the cover (2) .

2. The treatment apparatus according to the preceding claim, **characterized in that** the deflector (12) has an orientation surface (12a) of the gaseous medium toward the internal surface of the cover and onto which the gaseous medium entering the cover via the inlet (8) is directed.

3. The treatment apparatus according to claim 1, **characterized in that** the deflector **(**12₁) comprises a pipe with an elbow mounted on the inlet (8).

4. The treatment apparatus according to claim 3, **characterized in that** the deflector (12₁) comprises a pipe with an elbow mounted with the ability to rotate about its axis in order to regulate the orientation of the flow.

5. The treatment apparatus according to any one of the preceding claims, wherein the cover (2) is equipped with a viewing device (7) of the positioning zone (Z) for said individual's body part, and in that the flow control system (11) direct the gaseous medium toward the viewing device (7) so as to defog it.

6. The treatment apparatus according to any one of the preceding claims, **characterized in that** the inlet (8) and the outlet (9) are positioned side by side in divergent directions.

7. The treatment apparatus according to the preceding claim, **characterized in that** the inlet (8) and the outlet (9) are created on a body that is removable with respect to the cover.

8. The treatment apparatus according to any one of claims 5 to 7, characterized it comprises as viewing device (7) a window created in the cover or an optical system with or without image capture.

9. The treatment apparatus according to any one of the preceding claims, **characterized in that** the inlet (8) is designed to be connected to a source supplying liquid medium and **in that** the cover (2) is provided with a discharge outlet for the liquid medium corresponding to the outlet (9) for the gaseous medium or to an additional outlet (19) created in the cover.
